## Europäisches Patentamt

## European Patent Office

## Office européen des brevets

(11) Publication number: **0 255 100**
**A2**

(12) **EUROPEAN PATENT APPLICATION**

(21) Application number: 87110880.9

(22) Date of filing: 27.07.87

(51) Int. Cl.4: **A61K 31/505**

(30) Priority: 28.07.86 US 890048
17.07.87 US 71947

(43) Date of publication of application:
**03.02.88 Bulletin 88/05**

(84) Designated Contracting States:
**AT BE CH DE ES FR GB GR IT LI LU NL SE**

(71) Applicant: **WARNER-LAMBERT COMPANY**
**201 Tabor Road**
**Morris Plains New Jersey 07950(US)**

(72) Inventor: **Heifetz, Carl Louis**
**2640 Traver Road**
**Ann Arbor Michigan 48105(US)**

(74) Representative: **Mansmann, Ivo et al**
**c/o Gödecke AG - Patentabteilung Postfach**
**569 - Mooswaldallee 1-9**
**D-7800 Freiburg(DE)**

(54) **Use of trimetrexate and its salts for the preparation of pharmaceutical compositions for treating infections of the mycobacterium-avium-intracellulare complex.**

(57) Use of 5-methyl-6-[[(3,4-5-trimethoxyphenyl) amino] methyl]-2,4-quinazolinediamine (trimetrexate) and its pharmaceutically acceptable salts alone or in combination with an inhibitor of folic acid biosynthesis for the preparation of pharmaceutical compositions useful for treating infections of Mycobacterium avium-intracellulare complex in a patient.

EP 0 255 100 A2

## USE OF TRIMETREXATE AND ITS SALTS FOR THE PREPARATION OF PHARMACEUTICAL COMPOSI-
## TIONS FOR TREATING INFECTIONS OF THE MYCOBACTERIUM-AVIUM-INTRACELLULARE COMPLEX

The present invention relates to the use of trimetrexate for the preparation of pharmaceutical compositions for treating microbial infections in patients. More particularly, the present invention concerns the use of trimetrexate for preparing pharmaceutrical compositions for treating infections of the Mycobacterium avium-intracellulare complex.

Microorganisms of the Mycobacterium avium and Mycobacterium intracellulare groups are so similar that they are generally considered collectively (J. S. Chapman, The Atypical Mycobacteria and Human Mycobacteriosis, Plenum Medical Book Company, New York, 1970, p. 200). Throughout this specification, the general term MAI complex will be employed to denote the Mycobacterium avium-intracellulare complex. These organisms are distributed worldwide. Respiratory tract infections of the MAI complex occur throughout the United States, but the highest incidence of MAI complex related respiratory illnesses occur in the southeastern states. Infections are more common in rural populations, among miners, and patients with pneumoconioses, previous tuberculosis, and either restrictive or obstructive lung disease. Rapid progression of the infections is more likely in patients with severe underlying diseases or extensive infections at the time of initial diagnosis. Increasingly, infections are observed in patients having lowered resistance to respiratory tract infections due to a lowering of their infection defense mechanisms by acquired immune deficiency syndrome (AIDS). Generally, in most persons the symptoms are quite mild, especially in comparison with tuberculosis. However in patients having acquired immune deficiency syndrome, the infections are most often fatal.

Strains of M. avium and M. intracellulare vary widely in their in vitro susceptibility to chemotherapeutic agents. Many strains are highly resistant to most drugs which are effective anti-tubercular agents. Only some strains are susceptible in vitroto other antibacterial drugs such as the sulfonamides, erthromycin, amikacin, and a few cephalosphorins. As a result, current methods of treatment due to infections of the M. avium-intracellulare complex are often unsatisfactory (G. L. Mandell, R. G. Douglas, and J. E. Bennet, Ed's., Principles and Practices of Infectious Diseases, John Wiley & Sons, New York, 1984, p. 1419).

It is projected that within a few years the number of cases of acquired immune deficiency syndrome in the United States will rise above 200,000. In view of this troubling statistic, the seriousness of M. avium-intracellulare infections in AIDS patients, and the lack of generally effective chemotherapeutic agents for treating infections of the MAI complex in these and other patients, there is a need for an effective method of treating such infections.

In accordance with the present invention, there is provided the use of trimetrexate for the preparation of pharmaceutical preparations for treating infections of Mycobacterium avium-intracellulare complex in patients. The new pharmaceutical preparations contain an antimicrobially effective amount of trimetrexate<5-methyl-6-{[(3,4,5-trimethoxyphenyl)amino]methyl}-2-4-quinazolinediamine> alone or in combination with an inhibitor of folic acid biosynthesis.

5-methyl-6-{[(3,4,5-trimethoxyphenyl)amino] methyl}-2-4-quinazolinediamine, also known by the generic name trimetrexate, is an antifolate drug which hitherto has been used only for the treatment of psoriasis (cf. United States Patent 4,391,809), and malaria or mammalian neoplasms (cf. European Patent 0 051 415).

In accordance with the present invention it has been found that trimetrexate is bactericidal in vitro against the Mycobacterium avium-intracellulare complex of microorganisms.

The activity of trimetrexate at several concentrations ranging from 2 to 64 micrograms/ml was tested against fifteen strains of Mycobacteriumavium-intracellulare isolated from AIDS patients who were also suffering from infections of the MAI complex. Drug activity was measured using both a radiometric assay and growth inhibition analysis by the T100 method. (S.H. Siddiqi et al., J. Clin. Microbiol., 22: 919-923 (1985); S.H. Siddiqi et al., J. Clin. Microbiol., 13: 908-912 (1981); and D.E. Snyder et al., Am. Rev. Respir. Dis., 123: 402-406 (1981).

The radiometric assay is based upon the fact that the microorganisms metabolize $^{14}C$ labeled palmitic acid in Middlebrook 7H12 (BACTEC 12A) medium and release $^{14}CO_2$ which is measured in an automated ion chamber system (BACTEC 460, Johnston Laboratories, Towson, MD). A numerical value, referred to as the growth index is determined within the instrument.

Tests are performed by inoculating the organisms into vials of radiolabeled 7H12 medium, each containing an appropriate concentration of the test drug. For the drug-free control vial, the standard inoculum is diluted 1:100 so that growth in this vial represents 1% of the population added to each drug-containing vial. Interpretation of results is based on the amount of $^{14}CO_2$ produced in the presence and in the absence of drug.

The results of these tests employing trimetrexate at a concentration of 16 micrograms/ml against several strains of the Mycobacterium avium-intracellulare complex appear in Table 1. In addition, data are presented for the activity of a combination of trimetrexate at 16 micrograms/ml and 4,4'-sulfonylbisbenzenamine (dapsone) at 8 micrograms/ml. Dapsone is a sulfone compound which is a potent inhibitor of folic acid biosynthesis.

TABLE I

| MAI Complex Strain | Percent Microorganism Killed One Week Post | |
| --- | --- | --- |
| | TRIMETREXATE (16 µg/ml) | TRIMETREXATE + Dapsone (16 µg/ml) (8 µg/ml) |
| 100 | 65% | 59% |
| 101 | 68% | 67% |
| 108 | 70% | 53% |
| 109 | 42% | 78% |
| 116 | 91% | 60% |

As can be seen by the data presented in Table I, at these concentrations, trimetrexate alone or in combination with dapsone has potent bactericidal activity against all strains of the MAI complex which were tested.

In a second test, the minimal inhibitory concentration (MIC) required to inhibit 99% and 99.9% growth of the test strains of the Mycobacterium avium-intracellulare complex was determined employing the standard T100 method. The results appear in Table II where the MIC value is given for each of the tested MAI complex strains.

## TABLE II

| MAI Complex Strain | Approximate Minimal Inhibitory Concentration (MIC) (μg/ml) | |
|---|---|---|
| | 99% | 99.9% |
| 100 | 2 | 2 |
| 101 | 4 | 4 |
| 102 | 8 | 8 |
| 103 | 2 | 2 |
| 104 | 2 | 2 |
| 105 | 2 | 2 |
| 107 | 2 | 2 |
| 108 | 2 | 2 |
| 109 | 2 | 2 |
| 110 | 2 | 2 |
| 111 | 2 | 2 |
| 113 | 2 | 2 |
| 116 | 2 | 2 |
| 117 | 2 | 2 |
| 128 | 2 | 2 |

As can be seen by the data appearing in Table II, trimetrexate exhibited inhibitory activity against every strain of the MAI complex tested, at a concentration of 8 micrograms/ml or less.

The activity of trimetrexate against the MAI complex is aided by combining it with an inhibitor of folic acid biosynthesis as demonstrated by the data in Table 1 of the effect of a combination of trimetrexate and dapsone.

Trimetrexate is a potent inhibitor of the conversion of folic acid to tetrahydrofolic acid of the biochemical sequence p-aminobenzoic acid (PABA) > folic acid > tetrahydrofolic acid. Agents which inhibit the biosynthetic interconversion of PABA to folic acid thus aid the action of trimetrexate as an antibacterial agent by interrupting the folic acid metabolic pathway at a second point. The effect of this combination action can be seen in the data for strain 109 in Table I where trimetrexate alone resulted in a 42% kill of the strain, while the combination of trimetrexate with the folic acid biosynthesis inhibitor dapsone resulted in a 78% kill.

Thus, the present invention contemplates the use of trimetrexate both alone and in combination with inhibitors of folic acid biosynthesis for the treatment of infections of the Mycobacteriumavium-intercellulare complex. By the term "inhibitor of folic acid biosynthesis" as used throughout this specification and the appended claims is meant the class of sulfone, sulfonamide, and p-aminosalicylate drugs known to those skilled in the art to act by inhibiting the biochemical interconversion of PABA to folic acid.

By way of illustration, this class of inhibitors of folic acid synthesis includes sulfone drugs such as 4,4'-sulfonylbisbenzenamine (dapsone), [sulfonylbis-(1,4-phenyl-eneimino]bismethanesulfinic acid, disodium salt (sulfoxone sodium), 4,4'-diaminophenylsulfone-N,N-di(dextrose sodium sulfonate (glucosulfone), and N-[[5-amino-2-[(4-amino-phenyl)sulfonyl]phenyl]sulfonyl]acetamide, monosodium salt (acetosulfone sodium).

The class of inhibitors of folic acid synthesis which is contemplated by the present invention for use in combination with trimetrexate for the treatment of MAI complex infections also includes sulfonamide drugs such as 4-amino-N-2-pyrimidinylbenzenesulfonamide (sulfadiazine), 4-amino-N-(3,4-dimethyl-5-isoxazolyl)-benzenesulfonamide (sulfisoxazole), 4-amino-N-(5-methyl-3-isoxazolyl)benzene-sulfonamide (sulfamethoxazole), 4-amino-N-(1-ethyl-1,2-dihydro-2-oxo-4-pyrimidinyl)benzenesulfonamide (sulfacitine, 4-amino-N -(5-methyl-1,3,4-thiadiazol-2-yl)benzenesulfonamide (sulfamethizole), N'-(6-methoxypyridazin-3-yl)-sulfanilamide (sulfamethoxypyridazine), N'-(5-methoxy-pyrimidin-2-yl)sulfanilamide (sulfamethoxydiazine), N '-(3-methoxy pyrazin-2-yl)sulfanilamide (sulfametopyrazine), N'-(3,6-dimethoxy-pyrimidin-4-yl)sulfanilamide (sulfadimethoxine), and N'-5,6-dimethoxypyrimidin-4-yl)sulfanilamide (sulfadoxine).

Also contemplated by the present invention for use in combination with trimetrexate for the treatment of MAI complex infections is the compound p-aminosalicyclic acid and its pharmaceutically acceptable salts.

Trimetrexate forms pharmaceutically acceptable salts with organic and inorganic acids. Examples of suitable acids for salt formation are hydrochloric, sulfuric, phosphoric, gluconic, glucuronic, acetic, citric, oxalic, malonic, salicyclic, malic, fumaric, succinic, ascorbic, maleic, methanesulfonic, hydroxymethanesulfonic, 2-hydroxyethanesulfonic, and the like. Particularly preferred salts of trimetrexate useful in the medical method of this invention are the glucuronate and mono 2-hydroxyethanesulfonate salts. The salts are prepared by contacting the free base form with a sufficient amount of the desired acid in the conventional manner. The free base forms may be regenerated by treating the salt form with a base. For example, dilute aqueous base solutions may be utilized. Dilute aqueous sodium hydroxide, potassium carbonate, ammonia, and sodium bicarbonate solutions are suitable for this purpose.

For treating infections of the Mycobacterium avium-intracellulare complex, trimetrexate is administered, either alone or in combination with one of the above-named inhibitors of folic acid biosynthesis. Trimetrexate is administered, either orally in the form of a tablet or capsule or parenterally in sterile solution. Because of their increased solubility over the free base, salts of trimetrexate preferred for administration in the medical method of this invention. The preferred salt for oral administration is the isethionate (i.e., 2-hydroxyethanesulfonate) salt. For parenteral administration, the D-glucuronate salt is preferred.

A treatment regimen for a patient with MAI complex infection involves the administration of daily doses of from about 0.5 mg/m$^2$ of patient body area to about 240 mg/m$^2$. The patient is periodically tested to see if the MAI complex infection is still present and, if so, the treatment is continued.

If, in the opinion of the attending physician, the condition of the patient and/or the seriousness of the MAI complex infection merit the administration of higher doses of trimetrexate, it must be coadministered with an antidote to folic acid antagonists such as N-[4-[[(2-amino-5-formyl-1,4,5,6,7,8-hexahydro-4-oxo-6-pteridinyl)methyl]amino]benzoyl-L-glutamic acid, calcium salt (calcium leucovorin). For example, combination doses of from 30 mg/m$^2$ to 240 mg/m$^2$ per day of trimetrexate have been successfully coadministered with calcium leucovorin.

The exact dose and treatment regimen required for a particular patient will vary depending upon the prior medical history of the patient and the seriousness of the infection. The determination of the optimum dose and treatment regimen, however, is within the skill of the art. Generally, treatment is begun with lower dose, carefully increasing the dose until the desired affect is achieved.

Trimetrexate and its salts if desired in combination with adjuvants can be administered in a wide variety of oral and parenteral dosage forms. It will be obvious to those skilled in the art that the following dosage forms may comprise as the active component, either a compound, or a corresponding pharmaceutically acceptable salt of a compound, or a mixture of such compounds and/or salts.

For preparing pharmaceutical compositions from the compounds described by this invention, inert, pharmaceutically acceptable carriers can be either solid or liquid. Solid form preparations include powders, tablets, dispersable granules, capsules, cachets, and suppositories. A solid carrier can be one or more substances which may also act as diluents, flavoring agents, solubilizers, lubricants, suspending agents, binders, or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax, cocoa butter, and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions, suspensions, and emulsions. As an example may be mentioned water or water-propylene glycol solutions for parenteral injection. Liquid preparations can also be formulated in solution in aqueous polyethylene glycol solutions. Aqueous solutions suitable for oral use can be prepared by dissolving the active component in water and adding suitable colorants, flavors, stabilizing, and thickening agents as desired. Aqueous suspensions suitable for oral use can be made by dispersing the finely divided active component in water with viscous material, i.e., natural or synthetic gums, resins, methyl cellulose, sodium carboxymethyl cellulose, and other well-known suspending agents.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules, and powders in vials or ampoules. The unit dosage form can also be a capsule, cachet, or tablet itself or it can be the appropriate number of any of these packaged forms.

The quantity of active compound is a unit dose of preparation may be varied or adjusted from 1 mg to 100 mg according to the particular application and the potency of the active ingredient.

## Claims

1. Use of trimetrexate = <5-methyl-6-{[(3,4,5-trimethoxyphenyl)amino]methyl}-2,4-quinazolinediamine> or a pharmaceutically acceptable salt thereof alone or in combination with an inhibitor of folic acid biosynthesis for the preparation of pharmaceutical compositions for treating infections of Mycobacterium avium-intracellulare complex in a patient.

2. Use of trimetrexate according to Claim 1 wherein said pharmaceutically acceptable salt is selected from the 2-hydroxyethanesulfonate salt and the D-glucuronate salt.

3. Use of trimetrexate according to Claim 1 and 2, wherein 5-methyl-6-{[(3,4,5-trimethoxyphenyl) amino]methyl}-2,4-quinazolinediamine or a pharmaceutically acceptable salt thereof is provided in a dose ranging between about 0.5 mg/m² to about 240 mg/m² per day.

4. Use of trimetrexate in according with Claims 1 to 3, wherein said inhibitor of folic acid biosynthesis is a sulfone compound selected from the group consisting of 4,4′-sulfonylbisbenzenamine (dapsone), [sulfonylbis(1,4-phenyleneimino] bismethanesulfinic acid, disodium salt (sulfoxone sodium), 4,4′-diaminophenylsulfone-N,N-di(dextrose sodium sulfonate (glucosulfone), and N-[[5-amino-2-[(4-aminophenyl) sulfonyl]phenyl]sulfonyl]acetamide, monosodium salt (acetosulfone sodium).

5. Use of trimetrexate in accordance with Claims 1 to 3, wherein said inhibitor of folic acid biosynthesis is a sulfonamide compound selected from the group consisting of 4-amino-N -2-pyrimidinylbenzene sulfonamide (sulfadiazine), 4-amino-N-(3,4-dimethyl-5-isoxazolyl)benzenesulfonamide (sulfisoxazole), 4-amino-N-(5-methyl-3-isoxazolyl)-benzenesulfonamide (sulfamethoxazole), 4-amino-N-(1-ethyl-1,2-dihydro-2-oxo-4-pyrimidinyl)benzenesulfonamide (sulfacitine), 4-amino-N-(5-methyl-1,3,4-thiadiazol-2-yl)-benzenesulfonamide (sulfamethizole), N ′-(6-methoxypyridazin-3-yl)sulfanilamide (sulfamethoxypyridazine), N′-(5-methoxypyrimidin-2-yl)sulfanilamide (sulfamethoxydiazine), N′-(3-methoxypyrazin-2-yl) sulfanilamide (sulfametopyrazine), N′-(3,6-dimethoxypyrimidin-4-yl)sulfanilamide (sulfadimethoxine), and N′-5,6-dimethoxypyrimidin-4-yl)sulfanilamide (sulfadoxine).

6. Use of trimetrexate in accordance with Claims 1 to 3, wherein said inhibitor of folic acid synthesis is p-amino-salicylic acid or a pharmaceutically acceptable salt thereof.